# EUROPEAN PATENT APPLICATION

(11) **EP 4 360 521 A1**
(43) Date of publication of application: **01.05.2024**
(21) Application number: 21857012.5
(22) Date of filing: 10.09.2021
(51) Int. Cl.: A47K 7/04, A47K 1/04, A47K 10/48, A47K 5/12, A61L 2/10

(54) **HAND WASHING APPARATUS USING HIGH-PRESSURE WATER**

(30) Priority: 22.06.2021 KR 20210080892
(71) Applicant: CLEANM TECH INC., Saha-gu, Busan 49315 (KR)
(72) Inventor: KIM, Su Won, Changwon-si, Gyeongsangnam-do 51240 (KR)
(74) Representative: Dr. Gassner & Partner mbB
(86) International application number: PCT/KR2021/012348
(87) International publication number: WO 2022/270681

(57) **Abstract**

The present invention relates to a hand washing machine using high pressure water, and more specifically to a hand washing machine using high pressure water that can automatically wash person's hands and can remove bacteria without using components (e.g., a detergent) other than water. According to one embodiment of the present invention, the hand washing machine includes: a washing drum having a predetermined internal space; a first spray unit connected to the washing drum to spray washing water into the washing drum; a water tank connected to the first spray unit and storing predetermined washing water therein; a second spray unit connected to the washing drum to spray a cleaning agent into the washing drum; a cleaning agent tank connected to the second spray unit and storing a predetermined cleaning agent therein; a casing accommodating the washing drum, the water tank, and the cleaning agent tank; a hand entrance opening formed in the upper portion of the washing drum at one side of the casing; an air blowing unit blowing air in a direction toward the center of the hand entrance opening from the inner circumference of the hand entrance opening; a control unit transmitting control signals to the first spray unit, the second spray unit, and the air blowing unit; and an actuation means transmitting an actuation signal to the control unit. When the actuation means is operated, the hand washing machine is controlled through the steps of: operating the first spray unit to spray high pressure water for a predetermined time; operating the second spray unit to spray a cleaning agent for a predetermined time when it is determined that user's hands are not sensed in the washing drum after the water spray step; and operating the air blowing unit to blow air after the water spray step, such that the hands are washed with only the water sprayed in the water spray step.

## Description

### Technical Field

The present invention relates to a hand washing machine using high pressure water, and more specifically to a hand washing machine using high pressure water that can automatically wash person's hands and can remove bacteria without using components (e.g., a detergent) other than water.

### Background Art

Hand washing is currently proposed as one of the best ways to prevent highly contagious diseases such as COVID-19 and cold.

Since the outbreak of COVID-19, hand washing with hand sanitizers such as alcohol has become popular. However, according to studies conducted by many medical school professors, hand washing with only hand sanitizers is limited in its effectiveness and correct hand washing with soap for at least 30 seconds is effective in preventing the spread of infectious diseases such as COVID-19.

The hand washing survey (2019) conducted by the Korea Disease Control and Prevention Agency revealed that 1 out of 3 people do not wash their hands after going to the toilet and only 2.0% of people wash their hands properly. Touch objects with unwashed hands was found to be more susceptible to contamination with bacteria by about 56 times than that with washed hands.

It is necessary to correctly wash hands with running water for at least 30 seconds for personal health, but very few people practice it.

Under such circumstances, the present applicant has filed a patent application entitled "Cleaning Apparatus", which was then registered as Korean Patent No. 10-2165102. More specifically, the present applicant proposed a cleaning apparatus designed to wash hands with water and a cleaning agent wherein a wash casing is allowed to rotate in both directions such that hands are washed without leaving any unwashed areas.

However, the rotation of the washing casing in the cleaning apparatus causes noise and vibration and involves high energy consumption. Another limitation of the cleaning apparatus is that the apparatus needs considerable maintenance and repair work to protect its components such as a motor from damage or failure.

The use of cleaning agents for hand washing may cause skin troubles for people such as hospital medical staff who have to wash their hands dozens of times a day. Thus, there is a continuous need to wash hands with hypoallergenic cleaning agents or without using cleaning agents.

The present applicant has continued research and development on hand washing machines, and as a result, succeeded in developing a hand washing machine that can remove 99% of bacteria on hands with only water without using a cleaning agent. Thus, the present invention proposes a hand washing machine with improved performance such as washing power over the prior art.

### Detailed Description of the Invention

### Problems to be Solved by the Invention

The present invention has been made in an effort to overcome the above-described limitations of the prior art and an object of the present invention is to provide a hand washing machine using high pressure water that can remove 99% of bacteria on hands with only water without using any detergent.

### Means for Solving the Problems

The present invention has been made in view of the above-described limitations of existing hand washing machines and provides a hand washing machine using high pressure water, including: a washing drum 1 having a predetermined internal space; a first spray unit connected to the washing drum 1 to spray washing water into the washing drum 1; a water tank connected to the first spray unit and storing predetermined washing water therein; a second spray unit connected to the washing drum 1 to spray a cleaning agent into the washing drum 1; a cleaning agent tank connected to the second spray unit and storing a predetermined cleaning agent therein; a casing accommodating the washing drum 1, the water tank, and the cleaning agent tank; a hand entrance opening formed in the upper portion of the washing drum 1 at one side of the casing; an air blowing unit blowing air in a direction toward the center of the hand entrance opening 2 from the inner circumference of the hand entrance opening 2; a control unit transmitting control signals to the first spray unit, the second spray unit, and the air blowing unit; and an actuation means transmitting an actuation signal to the control unit, wherein when the actuation means is operated, the hand washing machine is controlled through the steps of: operating the first spray unit to spray high pressure water for a predetermined time; operating the second spray unit to spray a cleaning agent for a predetermined time when it is determined that user's hands are not sensed in the washing drum 1 after the water spray step; and operating the air blowing unit to blow air after the water spray step, such that the hands are washed with only the water sprayed in the water spray step.

The air blowing unit may include an air blower 3 accommodated in the casing and an air blowing nozzle 4 receiving air from the air blower 3 and accelerating the air in a direction parallel to an imaginary plane forming the hand entrance opening 2 such that the air is discharged at a flow rate higher than that at the discharge end of the air blower; and the casing may include a top cover 5 in which the hand entrance opening is formed and which has a shape to cover the air blowing unit and the washing drum 1 and discharge holes formed on the inner circumference of the hand entrance opening so as to correspond to discharge holes of the air blowing nozzle 4.

The washing drum 1 may be divided into a first group of layers having different heights and a second group of layers having heights different from those of the first group of layers and may include a plurality of first through holes 7 radially formed in the first group of layers and a plurality of second through holes 8 radially formed in the second group of layers; the first spray unit may include a water pump 9 receiving washing water from the water tank and supplying the washing water at a predetermined pressure, a water branch pipe distributing washing water supplied from the water pump 9, a plurality of water pipes 11 connected to the water branch pipe 10, and water nozzles 12 receiving washing water supplied through the water pipes 11 and spraying the washing water into the washing drum 1; the second spray unit may include a cleaning agent pump 13 receiving a cleaning agent from the cleaning agent tank and supplying the cleaning agent at a predetermined pressure, a cleaning agent branch pipe 14 distributing a cleaning agent supplied from the cleaning agent pump 13, a plurality of cleaning agent pipes 15 connected to the cleaning agent branch pipe 14, and cleaning agent nozzles 16 receiving a cleaning agent supplied through the cleaning agent pipes 15 and spraying the cleaning agent into the washing drum 1; the water pipes 11 may be introduced in pairs into each of the layers where the first through holes 7 are formed through one side of the layer and may be connected to the washing drum 1 while surrounding the layer from the one side, and pressure drops from the first water nozzle 12 to the last water nozzle 12 connected to each of the water pipes 11 may be distributed by the two water pipes 11; and the cleaning agent pipes 15 may be introduced in pairs into each of the layers where the second through holes 8 are formed through one side of the layer and may be connected to the washing drum 1 while surrounding the layer from the one side, and pressure drops from the first water nozzle 16 to the last water nozzle 16 connected to each of the cleaning agent pipes 15 may be distributed by the two cleaning agent pipes 15.

The washing drum 1 may have a predetermined cylindrical shape; the first through holes 7 may be formed in three layers; the second through holes 8 may be formed in two layers between the layers where the first through holes 7 are formed; the water nozzles 12 connected to the upper and middle layers where the first through holes 7 are formed may be inclined downward at angles of 40 to 50° to the horizontal; the water nozzles 12 connected to the lower layer where the first through holes 7 are formed may be directed horizontally; and the cleaning agent nozzles 16 may be directed horizontally.

The total number of the water nozzles 12 in the three layers may be 27, 9 per layer, and each water nozzle 12 may spray washing water conically at a divergence angle (θ) of 50 to 70°; and the total number of the cleaning agent nozzles 16 in the two layers may be 8, 4 per layer, and each cleaning agent nozzle 16 may spray a cleaning agent conically at a divergence angle (θ) of 110 to 130°.

The top cover 5 may include a sensing wall 20 having a predetermined cylindrical shape at a height higher than that of the hand entrance opening 2; the actuation means may include an infrared sensor 18 arranged on the sensing wall 20 at a height higher than that of the discharge holes 6 and a diffuse reflection pad 19 disposed opposite to the infrared sensor 18 on the sensing wall 20; and the water spray step may be performed after a waiting time of 1 second or more when a sensed value is received from the infrared sensor 18.

The hand washing machine may further include a drip tray 17 positioned under the washing drum 1 and having a size sufficient to completely accommodate the flat cross section of the washing drum 1 wherein the drip tray 17 includes a predetermined inclined plane 21 whose one side in the horizontal direction is lower in height than the other side and a wastewater pipe 22 through which wastewater collected at the lowest point of the inclined plane 21 is discharged; and the hand washing machine may further include a wastewater tank 23 accommodating wastewater discharged from the wastewater pipe 22 and a UV lamp irradiating ultraviolet light onto the wastewater tank 23.

The water spray step may be performed for 3 to 6 seconds so that 90% to 99.9% of bacteria on the skin of hands are removed by washing with water only.

According to a further embodiment of the present invention, the second spray unit, the cleaning agent tank, the cleaning agent spray step, and the second through holes 8 may be excluded from the hand washing machine using high pressure water.

### Effects of the Invention

According to one embodiment of the present invention, the hand washing machine can remove 99.9% of bacteria on hands with only water without using a cleaning agent.

In addition, the hand washing machine can provide convenience to users because the washing time is as short as 4 seconds.

In addition, the arrangements of the nozzles, the divergence angles, the spray angles, etc. can be appropriately adjusted such that hands are washed without leaving any unwashed areas, avoiding the need to rotate the washing drum.

In addition, the use of the diffuse reflection pad disposed opposite to the infrared sensor can prevent water from being wasted when malfunction of the sensor occurs.

In addition, the arrangement of water drainage holes in the machine can prevent operational failure of the machine caused by water leakage.

The use of the cleaning agent and the UV lamp to continuously sterilize the washing drum, the nozzles, and the wastewater tank can prevent the production of malodor and protect hands from contamination during washing.

The distribution and arrangement of the pipes in each of the layers can prevent the occurrence of pressure drops at the last nozzles connected to the pipes, achieving significant washing power (pressure) at all nozzles.

Furthermore, the blowing of high pressure air in the horizontal direction in the hand entrance opening during hand drying can provide convenience to users.

Moreover, the spray angles of the nozzles and the structural features (such as shape) of the hand entrance opening can be adjusted such that washing water is prevented from splashing out of the washing drum even when high pressure washing water is sprayed.

### Brief Description of the Drawings

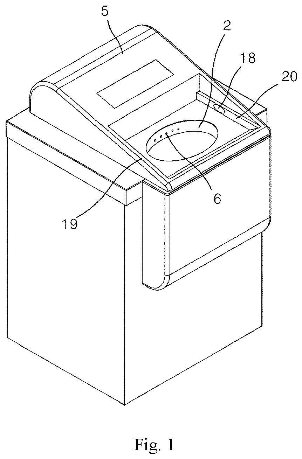

FIG. 1 is a perspective view of a hand washing machine using high pressure water according to one embodiment of the present invention.

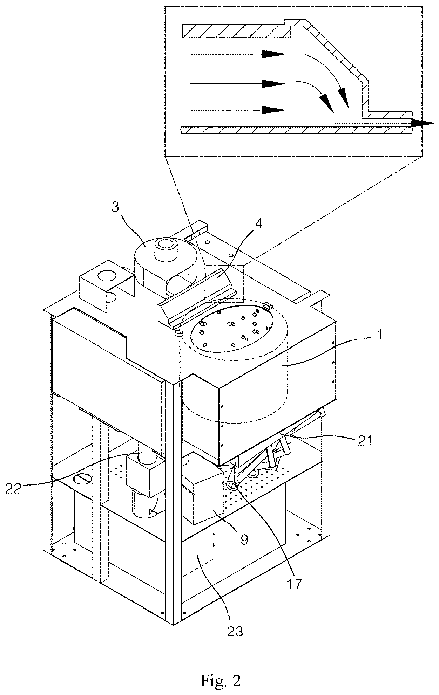

FIG. 2 is a perspective view of a hand washing machine using high pressure water without a casing according to one embodiment of the present invention.

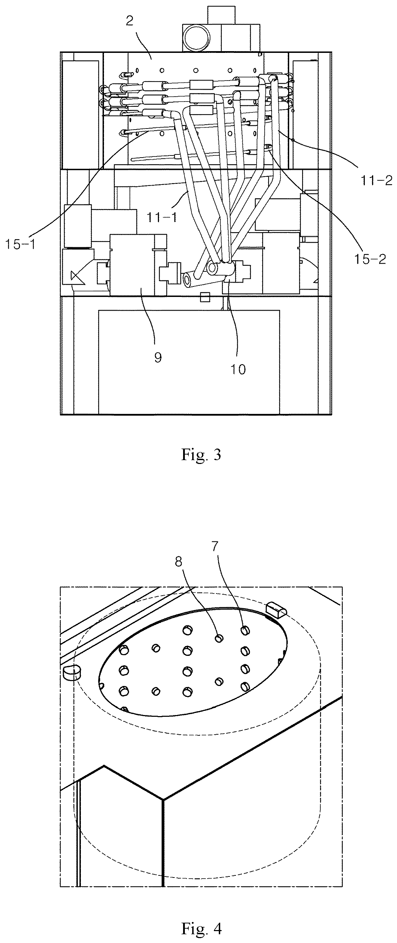

FIG. 3 illustrates the arrangement of pipes in a hand washing machine using high pressure water according to one embodiment of the present invention.

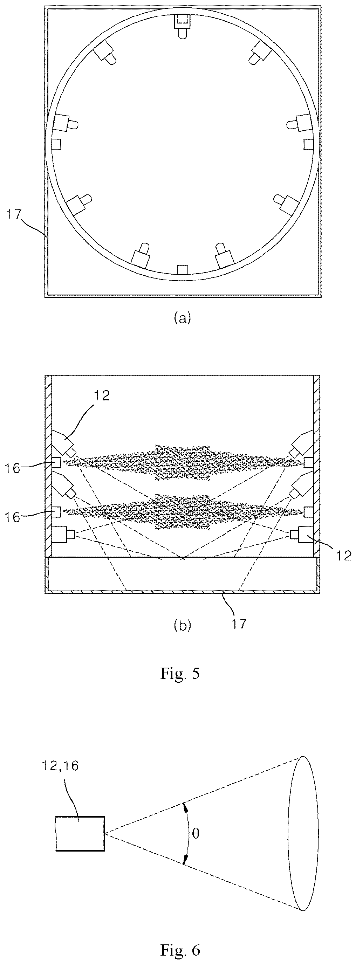

FIGS. 4 and 5 are detailed views of a washing drum of a hand washing machine using high pressure water according to one embodiment of the present invention.
FIG. 6 illustrates a spray pattern from a nozzle used in a hand washing machine using high pressure water according to one embodiment of the present invention.
FIGS. 7 to 9 show test data regarding the numbers of bacteria detected after use of a hand washing machine using high pressure water according to one embodiment of the present invention and after washing hands with alcohol, water, and soap.

### Mode for Carrying out the Invention

Various embodiments of the present disclosure will now be described with reference to the accompanying drawings. It should be understood, however, that the description is not intended to limit the present disclosure to particular embodiments and is construed as including various modifications, equivalents, and/or alternatives according to the embodiments of the present disclosure. In regard to the description of the drawings, like reference numerals refer to like elements.

The expressions "have," "may have," "include", "may include," "comprise," and "may comprise" as used herein indicates the presence of stated features (e.g., integers, functions, operations, elements or component) but do not preclude the presence of one or more other features.

The expressions "A or B", "at least one of A and/or B," or "one or more of A and/or B" as used herein may include all possible combinations of items listed together. For instance, the expression "A or B", "at least one of A and/or B," or "one or more of A and/or B" may indicate (1) including at least one A, (2) including at least one B or (3) including both at least one A and at least one B.

The expressions "1st", "2nd", "first", and "second" as used herein refer to modifying various components irrespective of the order and/or importance of the components. These expressions are only used to distinguish one component from another and are not intended to limit the corresponding components. For instance, both "a first user device" and "a second user device" indicate different user devices from each other irrespective of the order and/or importance of the devices. For example, a first component may be referred to as a second component and vice versa without departing from the scope of the present disclosure.

Terms used in the present disclosure are used only to describe specific embodiments and are not intended to limit the scope of other embodiments. The singular forms "a,", "an," and "the" are intended to include the plural forms as well, unless context clearly dictates otherwise. All technical and scientific terms used herein have the same meaning as commonly understood by those skilled in the art. Such terms as those defined in a generally used dictionary are to be interpreted to have the meanings equal to the contextual meanings in the relevant field of art, and are not to be interpreted to have ideal or excessively formal meanings unless clearly defined in the present disclosure. In some cases, terms defined in the present disclosure may not be interpreted as excluding embodiments of the present disclosure.

It is obvious that various modifications may be made by those skilled in the art, to which the present disclosure pertains without departing from the gist of the present disclosure, which is claimed in the claims, and such modifications should not be individually understood from the technical spirit or prospect of the present disclosure.

As used herein, the term "washing water" or "water" refers to water in a socially accepted sense and is intended to include mineral water, tap water, purified water, saline, and pure water (containing no foreign substances other than H₂O). Particularly, this term refers to water without components such as synthetic detergents and natural detergents that are specially added/mixed for hand washing. However, the addition of components such as flavoring agents (other than cleaning agents) can be optionally considered to fall within the range of equivalency of the claims.

One embodiment of the present invention provides a hand washing machine using high pressure water, including: a washing drum 1 having a predetermined internal space; a first spray unit connected to the washing drum 1 to spray washing water into the washing drum 1; a water tank connected to the first spray unit and storing predetermined washing water therein; a second spray unit connected to the washing drum 1 to spray a cleaning agent into the washing drum 1; a cleaning agent tank connected to the second spray unit and storing a predetermined cleaning agent therein; a casing accommodating the washing drum 1, the water tank, and the cleaning agent tank; a hand entrance opening formed in the upper portion of the washing drum 1 at one side of the casing; an air blowing unit blowing air in a direction toward the center of the hand entrance opening 2 from the inner circumference of the hand entrance opening 2; a control unit transmitting control signals to the first spray unit, the second spray unit, and the air blowing unit; and an actuation means transmitting an actuation signal to the control unit, wherein when the actuation means is operated, the hand washing machine is controlled through the steps of: operating the first spray unit to spray high pressure water for a predetermined time; operating the second spray unit to spray a cleaning agent for a predetermined time when it is determined that user's hands are not sensed in the washing drum 1 after the water spray step; and operating the air blowing unit to blow air after the water spray step, such that the hands are washed with only the water sprayed in the water spray step.

A UV lamp may be provided at one side of the washing drum. When the actuation means is not in operation, the UV lamp can be turned ON to sterilize the inside of the washing drum. Particularly, it is preferable to turn off the UV lamp when person's hands are positioned in the washing drum in the water spray step. This control is desirable in consideration of the adverse effect of UV light from the UV lamp on human skin.

The washing drum has a hollow shape. The washing drum has an open upper end through which user's hands enter and exit and an open lower end through which washing water is allowed to fall.

The casing functions to simply cover the internal components (e.g., the water tank and the control unit) from the outside. Accordingly, the case where the casing is excluded (or the case where the casing does not accommodate the washing drum, the water tank, and the cleaning agent tank) can also be considered to fall within the range of equivalency of the claims.

The hand entrance opening may be in the form of an elliptical through hole that has an area smaller than that of the washing drum to prevent high pressure washing water sprayed into the washing drum from splashing out of the washing drum.

For example, an infrared sensor, a switch or a foot switch may be used in the actuation means if needed.

The air blowing unit may include an air blower 3 accommodated in the casing and an air blowing nozzle 4 receiving air from the air blower 3 and accelerating the air in a direction parallel to an imaginary plane forming the hand entrance opening 2 such that the air is discharged at a flow rate higher than that at the discharge end of the air blower; and the casing may include a top cover 5 in which the hand entrance opening is formed and which has a shape to cover the air blowing unit and the washing drum 1 and discharge holes formed on the inner circumference of the hand entrance opening so as to correspond to discharge holes of the air blowing nozzle 4.

The air blowing unit can blow air in the horizontal direction to naturally remove water when a user removes his/her hands from the washing drum.

The washing drum 1 may be divided into a first group of layers having different heights and a second group of layers having heights different from those of the first group of layers and may include a plurality of first through holes 7 radially formed in the first group of layers and a plurality of second through holes 8 radially formed in the second group of layers; the first spray unit may include a water pump 9 receiving washing water from the water tank and supplying the washing water at a predetermined pressure, a water branch pipe distributing washing water supplied from the water pump 9, a plurality of water pipes 11 connected to the water branch pipe 10, and water nozzles 12 receiving washing water supplied through the water pipes 11 and spraying the washing water into the washing drum 1; the second spray unit may include a cleaning agent pump 13 receiving a cleaning agent from the cleaning agent tank and supplying the cleaning agent at a predetermined pressure, a cleaning agent branch pipe 14 distributing a cleaning agent supplied from the cleaning agent pump 13, a plurality of cleaning agent pipes 15 connected to the cleaning agent branch pipe 14, and cleaning agent nozzles 16 receiving a cleaning agent supplied through the cleaning agent pipes 15 and spraying the cleaning agent into the washing drum 1; the water pipes 11 may be introduced in pairs 11-1 and 11-2 into each of the layers where the first through holes 7 are formed through one side of the layer and may be connected to the washing drum 1 while surrounding the layer from the one side, and pressure drops from the first water nozzle 12 to the last water nozzle 12 connected to each of the water pipes 11 may be distributed by the two water pipes 11; and the cleaning agent pipes 15 may be introduced in pairs 15-1 and 15-2 into each of the layers where the second through holes 8 are formed through one side of the layer and may be connected to the washing drum 1 while surrounding the layer from the one side, and pressure drops from the first water nozzle 16 to the last water nozzle 16 connected to each of the cleaning agent pipes 15 may be distributed by the two cleaning agent pipes 15.

In the Korean patent issued to the present applicant, pipes are integrally formed but there is a limitation in that since pressure drops occur in nozzles connected to the ends of the pipes, a uniform (and strong) washing effect does not appear in all nozzles. Thus, the pipes are divided into two groups and arranged at left and right sides of the layers, respectively, in the hand washing machine of the present invention. This arrangement can minimize pressure differences and drops in the nozzles and is effective in uniformly and strongly washing all areas of hands without the need to rotate the washing drum.

The head of the water pump used in the hand washing machine of the present invention is preferably 7 m or more. In practice, when the pump has a head of 7 m, 99% of bacteria are removed by washing with water for 4 seconds, which was confirmed through repeated tests. More specifically, the water pump is selected from water pump products with motor outputs of 145 kW, 170 kW, and 182 kW, maximum flow rates of 45 LPM, 95 LPM, and 115 LPM, total heads of 7 m, 7.5 m, and 8 m, and a discharge diameter of 25 mm, which are minimum specifications for the hand washing machine of the present invention. In the description of the performances of the water pumps, the term "and" means any one of the performances.

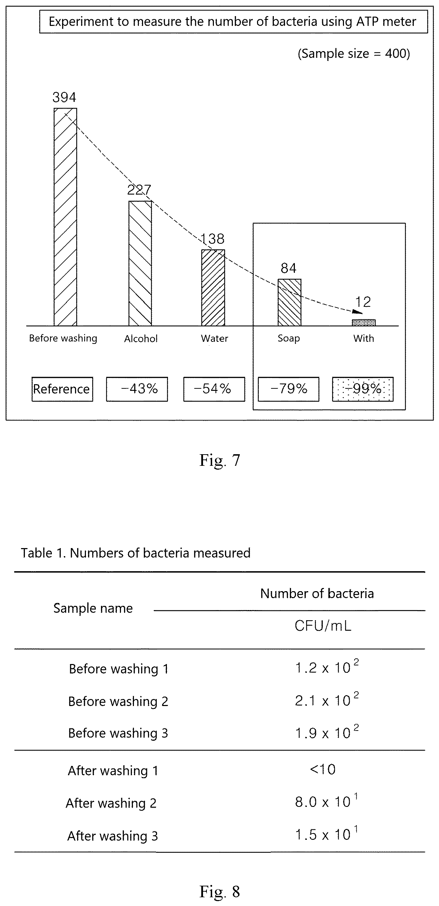

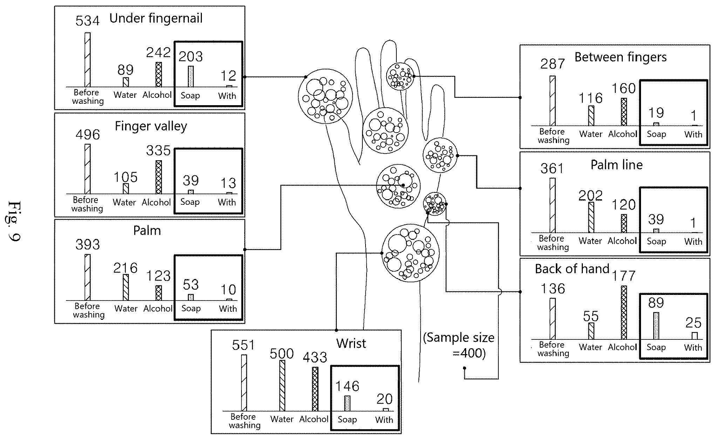

Here, it is preferable that washing water is sprayed at a rate of at least 0.8 L/s for 4 seconds. In one embodiment of the present invention, water is sprayed at a rate of 1 L/s (for example, in an amount of 4 L for 4 seconds). As a result, the test results shown in FIGS. 7 to 9 and Table 1 could be obtained. 99.9% of bacteria can be removed by optimizing the amount of water sprayed per second and/or the water spray time.

The washing drum 1 may have a predetermined cylindrical shape; the first through holes 7 may be formed in three layers; the second through holes 8 may be formed in two layers between the layers where the first through holes 7 are formed; the water nozzles 12 connected to the upper and middle layers where the first through holes 7 are formed may be inclined downward at angles of 40 to 50° to the horizontal; the water nozzles 12 connected to the lower layer where the first through holes 7 are formed may be directed horizontally; and the cleaning agent nozzles 16 may be directed horizontally.

The water nozzles connected to the upper and middle layers are preferably inclined at angles of 45° and their angles may be optionally adjusted in the range of 40° to 50°, as described above.

The arrangements and angle adjustments of the nozzles enable the concentration of the washing water spray on areas of user's hands entering predetermined positions of the washing drum.

The total number of the water nozzles 12 in the three layers may be 27, 9 per layer, and each water nozzle 12 may spray washing water conically at a divergence angle (θ) of 50 to 70°. The total number of the cleaning agent nozzles 16 in the two layers may be 8, 4 per layer, and each cleaning agent nozzle 16 may spray a cleaning agent conically at a divergence angle (θ) of 110 to 130°.

Within these divergence angle ranges, hands can be washed without leaving any unwashed areas, avoiding the need to rotate the washing drum.

More preferably, the divergence angle of the washing water is 60° and the divergence angle of the cleaning agent is 120°.

If the divergence angle of the washing water is larger than 60° and the divergence angle of the cleaning agent is larger than 120°, the spray pressures (speeds) may be rather lowered and the liquids sprayed from the adjacent nozzles may collide with each other, with the result that pressure losses occur, resulting in a further reduction in washing power. In one embodiment of the present invention, the hand washing machine is designed to maximize its washing power.

The top cover 5 may include a sensing wall 20 having a predetermined cylindrical shape at a height higher than that of the hand entrance opening 2; the actuation means may include an infrared sensor 18 arranged on the sensing wall 20 at a height higher than that of the discharge holes 6 and a diffuse reflection pad 19 disposed opposite to the infrared sensor 18 on the sensing wall 20; and the water spray step may be performed after a waiting time of 1 second or more when a sensed value is received from the infrared sensor 18.

For aesthetic and hygiene reasons, the top cover may be made of an ABS material. In the case where the top cover may be made of an ABS material with a glossy surface, the smooth surface of the top cover may cause malfunction of the infrared sensor. For example, even though human hands do not enter, a signal transmitted from the infrared sensor may be reflected from the sensing wall and received by the infrared sensor, causing malfunction of the infrared sensor. In one embodiment of the present invention, the pad is disposed opposite to the infrared sensor to cause diffuse reflection.

The diffuse reflection pad is preferably made of a black material with a rough surface. The diffuse reflection pad may be made by attaching a non-woven fabric, sandpaper or Velcro with a coarse surface to a black material that tends to absorb light. In this case, the diffuse reflection pad can absorb a signal from the infrared sensor and/or disperse the signal in all directions to prevent malfunction of the infrared sensor.

The hand washing machine may further include a drip tray 17 positioned under the washing drum 1 and having a size sufficient to completely accommodate the flat cross section of the washing drum 1 wherein the drip tray 17 includes a predetermined inclined plane 21 whose one side in the horizontal direction is lower in height than the other side and a wastewater pipe 22 through which wastewater collected at the lowest point of the inclined plane 21 is discharged; and the hand washing machine may further include a wastewater tank 23 accommodating wastewater discharged from the wastewater pipe 22 and a UV lamp irradiating ultraviolet light onto the wastewater tank 23.

In the Korean patent issued to the present applicant, the drip tray is designed in the form of a funnel but there is a limitation in that this design increases the overall height of the apparatus, causing inconvenience in use. In contrast, the drip tray used in the hand washing machine of the present invention is designed to be much lower in height than the funnel. This design is ergonomically advantageous because the overall height of the machine (especially, the height of the hand entrance opening) is adapted to the user's body size.

The water spray step is performed for 3 to 6 seconds so that 90% to 99.9% of bacteria on the skin of hands can be removed by washing with water only.

As described above, the hand washing machine of the present invention allows a user to wash his/her hands in a very short time while removing most bacteria on the hands. In the Korean patent issued to the present applicant, since a predetermined time is needed to keep the same washing effect, one or more units such as a unit playing music during washing are added. In contrast, the hand washing machine of the present invention does not cause inconvenience (for example, loss of emotional quality during waiting/use) to users even without music playing due to the short washing time. However, this does not mean the exclusion of a music playing unit from the hand washing machine of the present invention.

According to a further embodiment of the present invention, the second spray unit, the cleaning agent tank, the cleaning agent spray step, and the second through holes 8 may be excluded from the hand washing machine using high pressure water.

The expression "the second spray unit, the cleaning agent tank, the cleaning agent spray step, and the second through holes 8 may be excluded" means an embodiment in which one or more of the second spray unit, the cleaning agent tank, the cleaning agent spray step, and the second through holes 8 are excluded from an embodiment including the one or more of the second spray unit, the cleaning agent tank, the cleaning agent spray step, and the second through holes 8.

For example, this expression may be understood as an exclusion of the second spray unit, the cleaning agent tank, and the cleaning agent injection step from an embodiment in which the second through holes are not added.

FIGS. 7 to 9 and Table 1 present data from joint tests conducted by the Food Environment Research Center, Korea and the Hygiene and Microbiology Research Center, Japan.

Specifically, the numbers of bacteria detected after use of the hand washing machine according to the present invention (indicated by "With" in FIG. 7) were compared with those detected before hand washing and after washing hands with alcohol, water, and soap in FIGS. 7 to 9 and Table 1.

The numbers of bacteria in different areas of hands were measured before washing, after washing with alcohol, water, and soap, and after use of the hand washing machine. The results are shown in Table 1.

**[Table 1]**

| | Before washing | Water | Alcohol | Soap | Inventive | Removal rate (%) |
|---|---|---|---|---|---|---|
| Under fingernail | 534 | 89 | 242 | 203 | 12 | 97.8 |
| Finger valley | 496 | 105 | 335 | 39 | 13 | 97.4 |
| Palm | 393 | 216 | 123 | 53 | 10 | 97.5 |
| Wrist | 551 | 500 | 433 | 146 | 20 | 96.4 |
| Between fingers | 287 | 116 | 160 | 19 | 1 | 99.7 |
| Palm line | 361 | 202 | 120 | 39 | 1 | 99.7 |
| Back of hand | 136 | 55 | 177 | 89 | 25 | 81.6 |

| | | | | | | |
|---|---|---|---|---|---|---|
| * Since some subjects did not put their hands deep into the washing drum, the average number of bacteria remaining on the back of the subjects' hands was overestimated. | | | | | | |

The control unit may be provided with a predetermined communication means such as a Wi-Fi module for connection to a user terminal/management server such as a smartphone or a PC. Sensed values and operation signals from various sensors (including a water tank level sensor, a cleaning agent tank level sensor, a wastewater tank level sensor, and the infrared sensor) and the operation units (including the air blower, the water pump, the cleaning agent pump, the wastewater drainage pump, and the UV lamp) can be transmitted to the user terminal/management server.

A float level sensor may be employed as the water level sensor.

This construction imparts intelligent/smart/Internet of Things functions to the hand washing machine using high pressure water. The management server can monitor the levels in the fluid tanks (including the water tank, the cleaning agent tank, and the waste water tank) and can receive alarms when water and a cleaning agent are insufficient.

A water feeder may be connected to the water tank. The water feeder may be connected to a faucet. In this case, the amount of water supplied to the water tank can be automatically maintained constant by the water feeder, like when water is supplied to a washing machine/water purifier.

A wastewater drainage pump and a wastewater drainage pipe may be connected to the wastewater tank. In this case, wastewater can be drained into the sewer when accumulated above an appropriate level (when its level is above a predetermined value).

Sensed values from the sensors are accumulated and the accumulated data are learned so that the hand washing machine using high pressure water can be imparted with artificial intelligence functions.

The management server may collect data from hand washing machines using high pressure water that it manages. Such data include the number of times of operation per hour, water consumption, user's hand washing time, and sensed values from various sensors. The user's hand washing time can be measured when the user withdraws his/her hands from the hand entrance opening even though the water spray step is in progress. Washing times according to the places where the machines are located and the types of users are learned. If it is determined that the washing time is insufficient, the amount of water sprayed per second can be increased to increase the removal rate of bacteria.

For example, if it is determined that users who visit place A (those who use the machine installed in place A) at specific times wash their hands continuously for 2 seconds, the amounts of water sprayed may be weighted to 1.5 to 2 times higher levels than the default value.

### (Explanation of Reference Numerals)

1: Washing drum 2: Hand entrance opening
3: Air blower 4: Air blowing nozzle
5: Top cover 6: Discharge hole
7: First through hole 8: Second through hole
9: Water pump 10: Water branch pipe
11 (11-1: Left water pipe, 11-2: Right water pipe)
12: Water nozzle 13: Cleaning agent pump
14: Cleaning agent branch pipe
15 (15-1: Left cleaning agent pipe, 15-2: Right cleaning agent pipe)
16: Cleaning agent nozzle 17: Drip tray
18: Infrared sensor 19: Diffuse reflection pad
20: Sensing wall 21: Inclined plane
22: Wastewater pipe 23: Wastewater tank
θ: Divergence angle

## Claims

1. A hand washing machine using high pressure water, comprising: a washing drum having a predetermined internal space; a first spray unit connected to the washing drum to spray washing water into the washing drum; a water tank connected to the first spray unit and storing predetermined washing water therein; a second spray unit connected to the washing drum to spray a cleaning agent into the washing drum; a cleaning agent tank connected to the second spray unit and storing a predetermined cleaning agent therein; a casing accommodating the washing drum, the water tank, and the cleaning agent tank; a hand entrance opening formed in the upper portion of the washing drum at one side of the casing; an air blowing unit blowing air in a direction toward the center of the hand entrance opening from the inner circumference of the hand entrance opening; a control unit transmitting control signals to the first spray unit, the second spray unit, and the air blowing unit; and an actuation means transmitting an actuation signal to the control unit, wherein when the actuation means is operated, the hand washing machine is controlled through the steps of: operating the first spray unit to spray high pressure water for a predetermined time; operating the second spray unit to spray a cleaning agent for a predetermined time when it is determined that user's hands are not sensed in the washing drum after the water spray step; and operating the air blowing unit to blow air after the water spray step, such that the hands are washed with only the water sprayed in the water spray step.

2. The hand washing machine according to claim 1, wherein the air blowing unit comprises an air blower accommodated in the casing and an air blowing nozzle receiving air from the air blower and accelerating the air in a direction parallel to an imaginary plane forming the hand entrance opening such that the air is discharged at a flow rate higher than that at the discharge end of the air blower; and the casing comprises a top cover in which the hand entrance opening is formed and which has a shape to cover the air blowing unit and the washing drum and discharge holes formed on the inner circumference of the hand entrance opening so as to correspond to discharge holes of the air blowing nozzle.

3. The hand washing machine according to claim 1, wherein the washing drum is divided into a first group of layers having different heights and a second group of layers having heights different from those of the first group of layers and comprises a plurality of first through holes radially formed in the first group of layers and a plurality of second through holes radially formed in the second group of layers; the first spray unit comprises a water pump receiving washing water from the water tank and supplying the washing water at a predetermined pressure, a water branch pipe distributing washing water supplied from the water pump, a plurality of water pipes connected to the water branch pipe, and water nozzles receiving washing water supplied through the water pipes and spraying the washing water into the washing drum; the second spray unit comprises a cleaning agent pump receiving a cleaning agent from the cleaning agent tank and supplying the cleaning agent at a predetermined pressure, a cleaning agent branch pipe distributing a cleaning agent supplied from the cleaning agent pump, a plurality of cleaning agent pipes connected to the cleaning agent branch pipe, and cleaning agent nozzles receiving a cleaning agent supplied through the cleaning agent pipes and spraying the cleaning agent into the washing drum; the water pipes are introduced in pairs into each of the layers where the first through holes are formed through one side of the layer and are connected to the washing drum while surrounding the layer from the one side, and pressure drops from the first water nozzle to the last water nozzle connected to each of the water pipes are distributed by the two water pipes; and the cleaning agent pipes are introduced in pairs into each of the layers where the second through holes are formed through one side of the layer and are connected to the washing drum while surrounding the layer from the one side, and pressure drops from the first water nozzle to the last water nozzle connected to each of the cleaning agent pipes are distributed by the two cleaning agent pipes.

4. The hand washing machine according to claim 3, wherein the washing drum has a predetermined cylindrical shape; the first through holes are formed in three layers; the second through holes are formed in two layers between the layers where the first through holes are formed; the water nozzles connected to the upper and middle layers where the first through holes are formed are inclined downward at angles of 40 to 50° to the horizontal; the water nozzles connected to the lower layer where the first through holes are formed are directed horizontally; and the cleaning agent nozzles are directed horizontally.

5. The hand washing machine according to claim 4, wherein the total number of the water nozzles in the three layers is 27, 9 per layer, and each water nozzle sprays washing water conically at a divergence angle of 50 to 70°; and the total number of the cleaning agent nozzles in the two layers is 8, 4 per layer, and each cleaning agent nozzle sprays a cleaning agent conically at a divergence angle of 110 to 130°.

6. The hand washing machine according to claim 2, wherein the top cover comprises a sensing wall having a predetermined cylindrical shape at a height higher than that of the hand entrance opening; the actuation means comprises an infrared sensor arranged on the sensing wall at a height higher than that of the discharge holes and a diffuse reflection pad disposed opposite to the infrared sensor on the sensing wall; and the water spray step is performed after a waiting time of 1 second or more when a sensed value is received from the infrared sensor.

7. The hand washing machine according to claim 1, further comprising a drip tray positioned under the washing drum and having a size sufficient to completely accommodate the flat cross section of the washing drum wherein the drip tray comprises a predetermined inclined plane whose one side in the horizontal direction is lower in height than the other side and a wastewater pipe through which wastewater collected at the lowest point of the inclined plane is discharged; and further comprising a wastewater tank accommodating wastewater discharged from the wastewater pipe and a UV lamp irradiating ultraviolet light onto the wastewater tank.

8. The hand washing machine according to any one of claims 1 to 7, wherein the water spray step is performed for 3 to 6 seconds so that 90% to 99.9% of bacteria on the skin of hands are removed by washing with water only.

9. The hand washing machine according to claim 8, wherein the second spray unit, the cleaning agent tank, the cleaning agent spray step, and the second through holes are excluded.
